# EUROPEAN PATENT APPLICATION

(11) **EP 2 087 835 A1**
(43) Date of publication of application: **12.08.2009**
(21) Application number: 09447001.0
(22) Date of filing: 06.02.2009
(51) Int. Cl.: A61B 5/00, A61B 5/024, A61B 5/103, G08B 21/02

(54) **Fall detector**

(30) Priority: 08.02.2008 BE 200800073
(71) Applicant: Verhaert New Products And Services NV, 9150 Kruibeke (BE)
(72) Inventor: Verhaert, Koen, 2018 Antwerpen (BE)
(74) Representative: Van hunsel, Lieven M.S.

(57) **Abstract**

Fall detector, **characterised in that** it comprises a necklace (2) with a total length laying in a range of thirty up to eighty centimetres.

## Description

The present invention is related to a fall detector.

Various types of fall detectors are known and, beside the fall detection, some comprise one or more functions such as a movement detection, a heart pulsation detection or such.

In case where such a fall detector detects when the user is in need for assistance, for example in case a fall has been detected or when no movement at all is registered, such an alarm system will generate and send an alarm signal to a receiver such as an aid call centre or such.

Most of the well-known fall detectors are equipped such that they must be connected to a trousers belt.

A disadvantage thereof is however that such a fall detector cannot be applied in the case the concerning person using such a fall detector takes a shower, whereas most fall accidents take place exactly when entering or leaving the shower due to a slippery floor.

Moreover, such fall detectors connected to a trousers belt are not carried at night, as a result of which a person runs the risk to fall, for example at night when travelling in dark from the bed to the toilet, without being detected and signalled by means of an alarm signal.

Alternatively, also fall detectors are known which are provided of a wristband, such that they can easily be carried by the wrist.

However, such fall detectors with wristband show the disadvantage though that the arms make a lot of movements, both during the day and at night, as a result of which false alarms are often generated.

It is clear that the frequent occurrence of false alarms is undesirable, since this can lead the user to no longer use or put off the apparatus.

Another disadvantage of the well-known types of fall detectors exists in that they frequently show a too technical look and that they also lack discretion as a result of which elderly people are not tended to acquire or use such systems.

The present invention aims to offer a solution for one or more of the aforementioned and other disadvantages.

For that purpose, the present invention concerns a fall detector with the particular characteristics that it comprises a necklace with a total length in a range of twenty-five up to eighty centimetres, yet for adults generally larger than thirty centimetres, more in particular preferably in a range of thirty-five to fifty-five centimetres and still more preferably in a range of forty up to fifty centimetres.

With the total length of the necklace, the length is meant of the complete contour of this necklace (in closed status), measured along its length direction.

Such fall detector offers various advantages.

One of the advantages exists in the fact that the area around the neck region is by nature a relative stable area of the body, both during sleeping as during the normal day rhythm.

By keeping the total length of the necklace limited, also relative movements of the necklace with respect to the neck and/or the chest are limited or prevented.

As a consequence, the fall detector experiences few vibrations and/or shocks as a result of which the probability of related damages are limited.

Moreover, such a fall detector according to the invention results in a more accurate or cheaper fall detector.

Indeed, by limiting or preventing parasitic movements, also false alarms are prevented, which with usual fall detectors are frequently caused by such parasitic movements.

The prevention or strong reduction of parasitic movements also offers the possibility to apply a less complex processing of the signals, measured by the provided sensors and transmitted to the processor, and allows potentially to apply more simple or more compact sensors.

Still another advantage of such a fall detector according to the invention is that it can easily and discretely be carried around the neck, as it is provided of a necklace.

Since a necklace is carried directly on the body, it can also be carried when accessing a shower or when sleeping, and therefore also during the risky displacements between the bed and the toilet in dark.

Preferably, the fall detector according to the invention is made in the form of a jewel, which offers the advantage that it can be carried in a very discrete manner which makes the use of it acceptable.

According to a preferential characteristic of the invention, the fall detection means and optional movement detection means are linked with a processing entity which can be linked with alarm means.

Optionally, also a panic button is provided which is optionally also linked with the processing entity, and optionally the fall detector also comprises deactivation means to put off a false alarm.

A considerable improvement with respect to the existing systems is that the fall detector according to the invention offers the possibility to deactivate a falls alarm, by means of the above mentioned deactivation means.

According to a preferential characteristic of the invention, the aforementioned fall detector is provided of a hanger.

An advantage hereof is that, by the weight of the hanger, an additional stabilisation of the fall detector is obtained, which forms an additional measure against undesirable alarm messages.

With the aim of showing the characteristics of the present invention, hereafter are described, without any restrictive character and as an example, some preferred embodiments of a fall detector according to the invention, with reference to the enclosed drawings, where:
figure 1 represents schematically and in perspective a fall detector according to the invention;
figure 2 represents on a larger scale the part which in figure 1 has been indicated with F2;
figure 3 represents a variant of fall detector according to figure 1;
figure 4 represents on a larger scale the part which in figure 3 is indicated with F4.

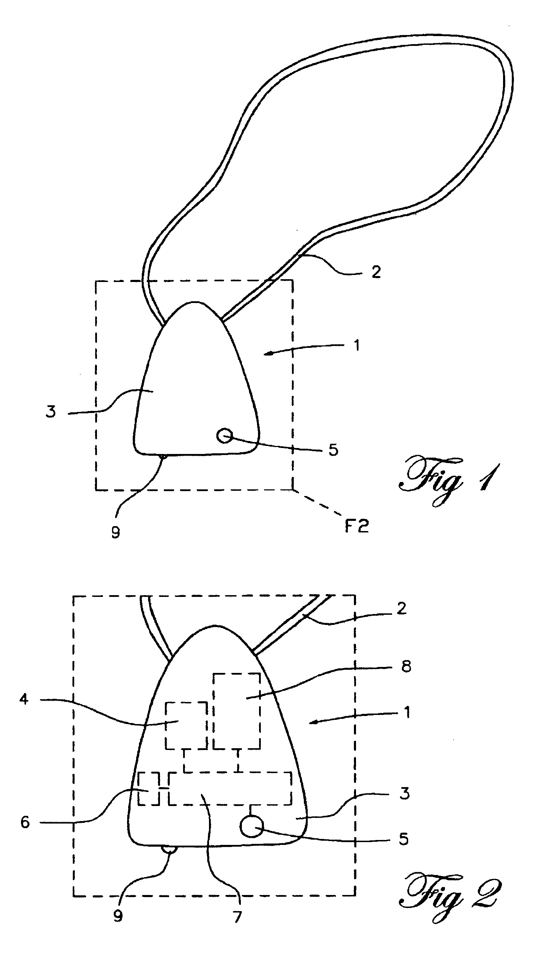

Figures 1 and 2 represent a fall detector 1 according to the invention which in the given case is made in the form of a jewel and which is, according to the invention, provided with a necklace 2 to which in this case a hanger 3 is connected, which as an example shows the look of a stone.

Preferably the necklace shows 2 a restricted length and, according to the invention, it shows a total length which is in a range of thirty up to eighty centimetres, or rather still in a range of thirty-five up to fifty-five centimetres, or still more preferably in a range of forty up to fifty centimetres.

With the total length of the necklace, the length is meant of the complete contour of this necklace 2 (in closed status), measured along its length direction.

The fall detector 1 is provided of fall detection means 4 and according to a preferred characteristic of the invention, also of a panic button 5 and/or of movement detection means 6, each linked to a processing entity 7 which can be connected to alarm means 8.

In the example of figure 1, the fall detector 1 comprises besides the fall detection means 4, also a panic button 5 and movement detection means 6, yet, this is according to the invention not required, since the fall detector 1 can include for example only one of these means 4 and 6, where the panic button is entirely optional.

Each of the aforementioned fall detection means 4, the panic button 5, the movement detection means 6, the processing entity 7 and the alarm means 8 are, in this case but necessarily, provided in the aforementioned hanger 3 and are for example fed by a battery, not represented in the figures, and preferably also provided in said hanger 3 and which optionally can be replaced or which optionally is rechargeable.

The aforementioned fall detection means 4 can for example be provided in a well-known manner of an accelerometer which can detect a change in the direction of the gravitation and which is also capable of measuring accelerations and an impact.

The panic button 5 is in this case made in the form of a push button, yet, it can according to the invention also be made in a lots of ways, as for example in the form of a switch, a pressure sensor or other appropriate switching means which allow a user, in case of urgency, to generate an alarm signal.

According to the invention, the fall detector 1 also includes deactivation means for putting off a false alarm, which deactivation means in this case are also formed by the aforementioned panic button 5, yet, which can also be made in the form of separate switching means.

The movement detection means 6 have in this case been designed with the aim of measuring an optionally occurring inactivity and for example also comprise an accelerometer.

The movement detection means 6 can thus also be considered as inactivity detection means.

The processing entity 7, also called processor, can for example be configured in the form of an integrated circuit (IC).

The alarm means 8 can according to the invention be configured in a variety of manners and consist in this case of a communication entity with a sender which can forward an alarm signal to a receiver which is optionally situated in the immediacy of the fall detector 1, or at a distant thereof.

Finally, the fall detector 1 is in this case also provided of indication means 9 which in this example are configured in the form of a so-called light emitting diode (LED), yet, which also can be realised in a variety of manners, such as in the form of a buzzer, a trembler, a siren or such.

The use and the functioning of a fall detector 1 according to the invention are very simple and as follows.

During the use, the fall detector 1 according to the invention, is carried around the neck of a user by means of the aforementioned necklace 2, as a result of which a stable situation of this fall detector 1 is obtained.

When the user of the fall detector 1 falls, this event is detected by the aforementioned fall detection means 4 which give a signal to the aforementioned processing entity 7.

The processing entity 7 triggers for its part the alarm means 8 in an appropriate manner, such that these alarm means 8 generate an alarm signal that is sent to an appropriate receiver not represented in the figures.

The alarm means 8 can for example be provided of a communication entity which comprises a sender able to send a signal over a wireless communication network.

In this case, the alarm signal generated by the alarm means 8 can be sent for example to an emergency centre of aid services or to any other receiver apparatus of a care provider.

As such it is according to the invention possible that the aforementioned alarm signal exists of an SMS or a voicemail which is sent to the GSM apparatus of a correspondent preprogrammed in the alarm means 8, such as a doctor, a nurse, a family member, a friend, a neighbour or such.

By means of the aforementioned deactivation means it is according to the invention possible to put off a false alarm.

For the represented example of fall detector 1 in figures 1 and 2, it can be realised by having the processing entity 7, at the detection of a fall by the fall detection means 4, first to wait a certain interval of time before triggering the alarm means 8.

During this interval of time, which for example can amount to fifteen seconds, the aforementioned indication means 9 are signalled by the processing entity 7, such that the user is informed of the fact that an alarm signal will be sent.

In this case the concerning indication happens by lighting the LED, yet, by preference also a sound signal is created such as a buzz tone or a beep tone.

If the user activates the deactivation means during the concerning time interval, which in this case is obtained by pressing the panic button 5, the processing entity 7 will not trigger the alarm means 8 and no alarm signal will be sent.

If the user however does not apply the deactivation means during the aforementioned interval, the processing entity 7 as described above, will activate the alarm means 8.

In an analogous manner as described here above, an alarm signal can also be generated when the aforementioned movement detection means 6 identify no movement of the user of the fall detector 1 during a certain time interval.

A third possibility for activating an alarm signal consists in the impression of the aforementioned panic button 5.

Also in this last two cases, the user can eliminate a false alarm by means of the aforementioned deactivation means in the manner as described above.

Preferably, the hanger 3 is made heavy such that a more stable position of this hanger 3 is obtained during the use and the swinging of it is reduced, so that the chance of a false alarm is strongly reduced.

Thanks to the fact that the necklace 2 is made relatively short, an additional stabilising affect is obtained, as a result of which also the occurrence of erroneous indication of a fall is avoided.

Alternatively or additionally, the fall detector 1 can be stabilised by making the necklace 2 of a relatively rigid material.

The shape design of the necklace 2 and/or of the hanger 3 is preferably such that, in case of a fall where the person continues lying on the ground, the hanger 3 cannot remain occasionally in a vertical position which could prevent the detection of the fall by the fall detection means 4.

This can for example be realised by having the necklace 2 introduced through a tubular opening in the hanger 3 or by not having a flat but rounded base of the hanger 3, such that the hanger 3 cannot stand vertically on a subsoil.

Since in this case, the fall detector 1 is made in the form of a jewel, the use of other jewels around the neck is made superfluous, as a result of which costs are saved.

Since the fall detector 1 according to the invention comprises a necklace 2, the orientation of this fall detector 1 can be used as direct indication for the orientation of the user, in contrast to traditional fall detectors which are for example applied around the wrist.

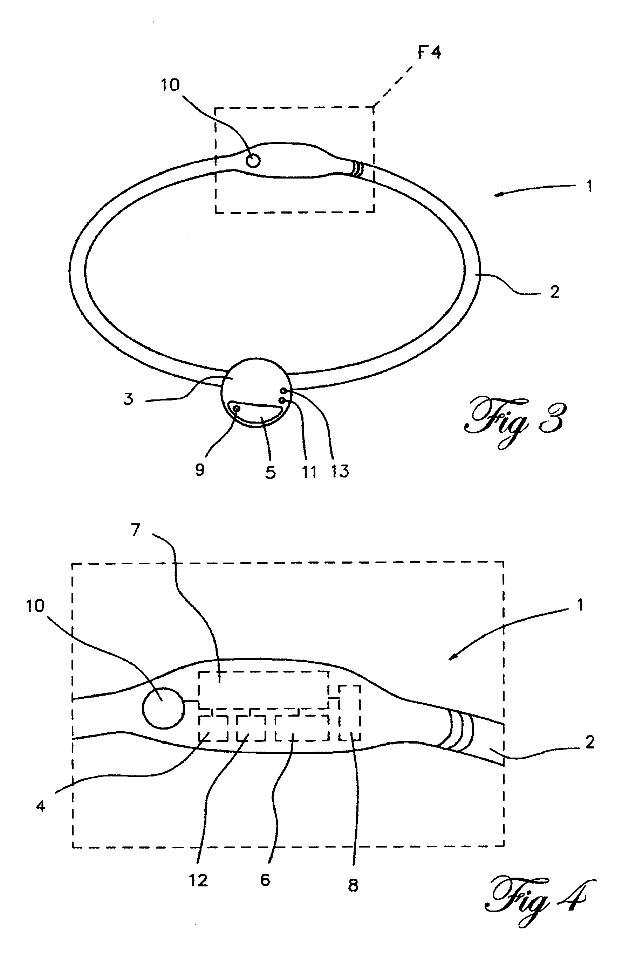

In the figures 3 and 4 represents an alternative of a fall detector 1 according to the invention, which is mainly constructed in the same manner as the embodiment from the figures 1 and 2.

In this example, the aforementioned fall detection means 4, the movement detection means 6, the processing entity 7 and the alarm means 8 have all been introduced in the necklace 2 and more particularly at the height of the neck zone of this necklace 2 which during use of the fall detector 1 is at all time positioned at the neck of the user.

In this case, the fall detector 1 is also provided of a temperature sensor 10 for measuring the body temperature of a user, which temperature sensor 10 is in this case also provided at the neck zone of the necklace 2.

The panic button 5 and the indication means 9 are in this case however provided in the hanger 3.

Furthermore, the fall detector 1 comprises in this case a signal light 10 that indicates when the battery of the processing entity 7 must be replaced, where in this case this signal light 10 is also situated on the hanger 3.

According to a preferred characteristic of the invention, the fall detector 1 is also provided with a receiver 12 which is connected to the aforementioned processing entity and which enables a two-way communication between the fall detector 1 and a receiver.

For that purpose, the fall detector 1 preferably also includes a confirming indication 13, for example in the form of a blinking light or such which is also provided on the hanger 3.

It speaks for itself that the aforementioned receiver 12 can also be made as a one-piece unit with the aforementioned sender of the communication entity of the alarm means 8, resulting in one common sender/receiver.

The functioning of such embodiment of a fall detector 1 according to the figures 3 and 4 is analogous to the functioning of the embodiment of the figures 1 and 2.

In this case however, an additional alarm signal can be generated at the detection of a decreasing or rising body temperature of the user.

Since the temperature sensor 10 is situated in the neck of the necklace 2, the temperature sensor 10 is at all time in contact with the skin of the user, as a result of which a good measurement of the body temperature is possible.

In the case the battery, which feeds the processing entity 7, must be replaced, the aforementioned indication light 11 will indicate so.

A particular characteristic of this embodiment of fall detector 1 is that it allows to set up a two-way communication by means of the thereto provided sender of the alarm means 8 and the receiver 12, where a care worker is given the chance, after having received an alarm signal from a fall detector 1, to send a confirmation signal to this fall detector 1 to indicate that they have received the message.

This is possible, in the represented example of the figures 3 and 4, because the processing system, when receiving the confirmation signal via the receiver 12, will activate the confirmation indication 13, in this case by lighting the blinking light.

According to a preferred characteristic of the invention, not represented in the figures, the fall detector 1 is also provided of a neck pulsation detector which preferably, yet not necessary, is provided in the aforementioned hanger 3, yet, the neck pulsation detector can for example also be provided in the necklace 2.

Since the fall detector 1 comprises a necklace 2, it can be configured such that the concerning hanger 3 and the therein provided neck pulsation detector are situated at the height of the heart or in the immediacy of a neck vein of the user, as a result of which the heart pulsation of this user can be measured well.

In each of the above described embodiments of the fall detector 1, use is made of a hanger 3. According to the invention, the presence of such a hanger 3 is though not required. However, an appropriate coordination of the weight of the hanger 3 and the rigidity of the necklace 2, and depending on the position of the sensors, the stability can be optimised.

It is clear that the length of the necklace 2 can be tuneable and adaptable.

The current invention is by no means restricted to the embodiments described and represented in the figures, which embodiments are only given as an example, yet a fall detector according to the invention can be realised in numerous forms and dimensions, without falling outside the framework of the invention.

## Claims

1. Fall detector, **characterised in that** it comprises a necklace (2) with a total length laying in a range of thirty up to eighty centimetres.

2. Fall detector according to claim 1, **characterised in that** the necklace (3) shows a total length laying in a range of thirty-five up to fifty-five centimetres.

3. Fall detector according to claim 1 or 2, **characterised in that** the necklace (3) shows a total length laying in a range of forty up to fifty centimetres.

4. Fall detector according to any of the previous claims, **characterised in that** it is made in the form of a jewel.

5. Fall detector according to any of the previous claims, **characterised in that** it is provided of a hanger (3).

6. Fall detector according to any of the previous claims, **characterised in that** the fall detection means (4) and the optional movement detection means (6) and/or an optional panic button (5) are linked with a processing entity (7) which for its part can be linked with alarm means (8) and **in that** it also can comprise deactivation means for putting off a false alarm.

7. Fall detector according to any of the previous claims, **characterised in that** it is provided of a temperature sensor for measuring the body temperature of a user.

8. Fall detector according to claim 7, **characterised in that** the aforementioned temperature sensor is provided at the height of the neck zone of the necklace (2).

9. Fall detector according to any of the previous claims, **characterised in that** it is provided of a heart pulsation detector.

10. Fall detector according to claims 5 and 9, **characterised in that** the aforementioned heart pulsation detector is provided in the aforementioned hanger (3).

11. Fall detector according to claim 6, **characterised in that** the aforementioned fall detection means (4) include an accelerometer which is provided at the height of the neck zone of the aforementioned necklace (3).

12. Fall detector according to claim 6, **characterised in that** it is provided of a battery for feeding the aforementioned processing entity (7).

13. Fall detector according to claims 5 and 6, **characterised in that** the aforementioned processing entity (7) and alarm means (8) are provided in the aforementioned hanger (3).

14. Fall detector according to claim 6, **characterised in that** it is also provided of a sender and/or a receiver which is connected to the aforementioned processing entity (7).

15. Fall detector according to claim 6, **characterised in that** the aforementioned deactivation means and the aforementioned panic button are integrated.
